# EUROPEAN PATENT APPLICATION

(11) **EP 4 616 723 A1**
(43) Date of publication of application: **17.09.2025**
(21) Application number: 23888830.9
(22) Date of filing: 31.05.2023
(51) Int. Cl.: A23L 7/104, A23L 29/00, A23L 5/10, A23L 3/36, C12N 1/20, C12R 1/07

(54) **GRAIN-FERMENTED ENZYME HAVING GLUTEN-DEGRADING ABILITY, METHOD FOR PRODUCING SAME, AND FOOD COMPOSITION COMPRISING SAME**

(30) Priority: 10.11.2022 KR 20220149272
(71) Applicant: NPK INC., Jeollanam-do 57309 (KR)
(72) Inventor: SHON, Sung Oh, Gwangju 61092 (KR); JI, Hwee Won, Gwangju 61203 (KR)
(74) Representative: Schweitzer, Klaus
(86) International application number: PCT/KR2023/007441
(87) International publication number: WO 2024/101561

(57) **Abstract**

The present invention relates to a grain-fermented enzyme which has α-amylase activity, protease activity, gluten degradation activity and fibrinolytic enzyme activity by fermenting a grain with a *Bacillus amyloliquefaciens* strain; a method for producing the same; a food composition including the same; and a novel *Bacillus amyloliquefaciens* strain having a gluten degradation ability. According to the present invention, no food additive enzyme is added, and thus, there is the advantage of providing a grain-fermented enzyme having excellent taste and economic efficiency and a gluten degradation ability.

## Description

### [Field of Invention]

The present invention relates to a grain-fermented enzyme having gluten degradation ability, a method for producing the same, and a food composition including the same, and more specifically, to a grain-fermented enzyme having α-amylase activity, protease activity, gluten degradation activity and fibrinolytic enzyme activity obtained by fermenting grains with *Bacillus amyloliquefaciens* strain, a method for producing the same, a food composition including the same, and novel *Bacillus amyloliquefaciens* strains having α-amylase activity, protease activity, gluten degradation activity and fibrinolytic enzyme activity.

### [Background of Invention]

One of the most important elements of our body is enzymes. The enzymes are necessary to properly perform our body functions, and if the enzymes do not exist in the body, many body functions such as breathing, digestion, and nutrient synthesis may be lost.

When our body is young, it can synthesize and obtain abundant enzymes within the body. However, modern people's bad eating habits, such as smoking, drinking, high fat, and excessive sugar intake, may cause stress to cells, kill them and reduce enzyme synthesis in the body, and may force remaining cells to produce more digestive enzymes, which in turn, ultimately repeats a vicious cycle of deteriorating durability.

Therefore, by taking enzyme supplements to improve digestion, it may help decompose the ingested food and reduce enzyme synthesis in the body, which can not only maintain gastric health but also improve circadian rhythm.

"Enzyme food" refers to a product that contains a large amount of enzymes by cultivating edible microorganisms in vegetable raw materials, includes enzyme-containing parts extracted from food, or is a product of processing the extracted part as a main ingredient.

These enzyme foods are known to contain various trace elements and physiologically active substances that help digestion and absorption through the production of various physiologically active substances and nutrients and the proliferation of beneficial bacteria according to the fermentation and maturation process of the food's own enzymes and microorganisms.

Grains fermented enzyme is a generic term for enzyme foods made by cultivating edible microorganisms in grain raw materials so as to contain a large amount of enzymes. In this regard, as the grain raw materials, any one grain among brown rice, soybeans, barley, and mixed grains (wheat, corn, and adlay) may be used (Korean Patent Registration Publication No. 10-1855125). Further, the prior art is disclosed in that grains such as rice, barley species such as barley, wheat, rye, and oat, or mixed grains such as millet, maize, foxtail millet, Japanese millet, buckwheat, and adlay are used (Korean Patent Registration Publication No. 10-2088758).

However, as in the present invention, a grain-fermented enzyme having α-amylase activity, protease activity, gluten degradation activity and fibrinolytic enzyme activity, which is produced by fermenting grains with *Bacillus amyloliquefaciens* strain, is still not known in the art.

### [Prior Art Document]

### [Patent Document]

(Patent Document 1) Korean Patent Registration Publication No. 10-1855125
(Patent Document 2) Korean Patent Registration Publication No. 10-2088758

### [Summary of Invention]

### [Technical Problem to be Solved]

An object of the present invention is to provide a grain-fermented enzyme having gluten degradation ability by fermenting grains with Bacillus strains.

Another object of the present invention is to provide a method for producing a grain-fermented enzyme having gluten degradation ability by fermenting grains with Bacillus strains.

In addition, another object of the present invention is to provide a food composition which includes a grain-fermented enzyme having gluten degradation ability by fermenting grains with Bacillus strains.

Further, another object of the present invention is to provide a novel *Bacillus amyloliquefaciens* strain used for the production of a grain-fermented enzyme having gluten degradation ability.

### [Technical Solution]

In order to achieve the above objects, the present invention provides a grain-fermented enzyme fermented by *Bacillus amyloliquefaciens* strain, wherein the grain is one or more selected from the group consisting of defatted soybean, oat, brown rice, white bean, barley, sorghum, linseed, rice germ, pea, pea pod, wheat, rye and millet.

In one embodiment of the present invention, the strain may be *Bacillus amyloliquefaciens* NPKE6 deposited under accession number KCCM13257P.

In one embodiment of the present invention, the grain-fermented enzyme may have α-amylase activity, protease activity, gluten degradation activity and fibrinolytic enzyme activity.

In order to achieve the above objects, the present invention includes a method for producing a grain-fermented enzyme including: the steps of (a) soaking grains in water; (b) removing the soaking water; (c) steaming the grains, and then sterilizing them; (d) subdividing the sterilized grains, and then cooling them; (e) inoculating the subdivided grains with *Bacillus amyloliquefaciens* strain, and then mixing them; and (f) fermenting the grain inoculated with the strain.

In one embodiment of the present invention, the method for producing a grain-fermented enzyme may further include the step of hot air drying and powdering after step (f).

In one embodiment of the present invention, the grain may be one or more selected from the group consisting of defatted soybean, oat, brown rice, white bean, barley, sorghum, linseed, rice germ, pea, pea pod, wheat, rye and millet.

In one embodiment of the present invention, the strain may be *Bacillus amyloliquefaciens* NPKE6 deposited under accession number KCCM13257P.

In order to achieve the above objects, the present invention provides a food composition including the grain-fermented enzyme.

In one embodiment of the present invention, the food composition may be an enzyme food.

In order to achieve the above objects, the present invention provides *the Bacillus amyloliquefaciens* NPKE6 strain deposited under the accession number KCCM13257P.

In one embodiment of the present invention, the strain may have α-amylase activity, protease activity, gluten degradation activity and fibrinolytic enzyme activity.

### [Effect of Invention]

According to the present invention, there is an advantage of providing a grain-fermented enzyme that is economically excellent and has α-amylase activity, protease activity, gluten degradation activity and fibrinolytic enzyme activity.

Further, according to the present invention, since only the grain-fermented enzyme and vegetable raw materials are added, it complies with the recommendations of the Ministry of Food and Drug Safety, and using the grain raw materials may have the advantage of providing a health-oriented superfood.

### [Brief Description of Drawings]

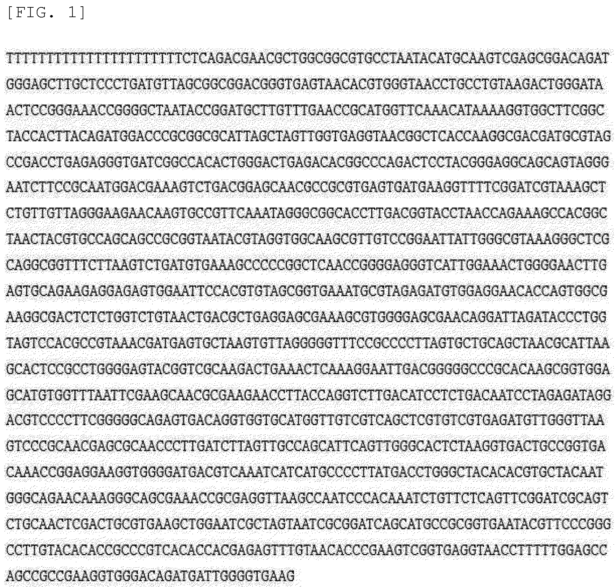

FIG. 1 shows the 16S rRNA base sequence of the *Bacillus amyloliquefaciens* NPKE6 strain (KCCM13257P) of the present invention.

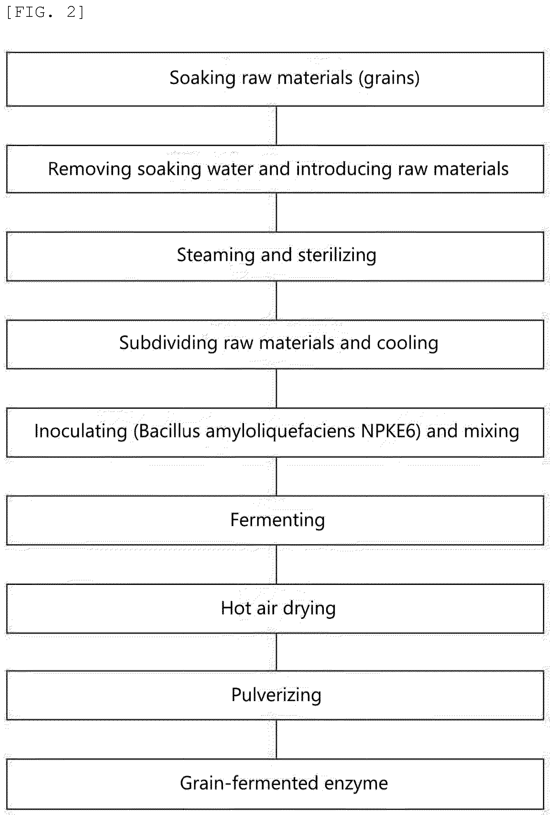

FIG. 2 shows a process diagram of the manufacturing of a grain-fermented enzyme according to an embodiment of the present invention.
FIG. 3 shows results of checking the transparency 24 hours after inoculating the strain into an aqueous solution of 0.1% glucose and 0.5% gluten in order to confirm the gluten degradation ability of the NPKE6 strain according to the present invention.
FIG. 4 shows results of confirming the gluten degradation ability of *Bacillus amyloliquefaciens* NPKE6 strain using the Gliadin kit.
FIG. 5 shows results of checking the transparency 24 hours after inoculating the strain filtrate and the grain-fermented enzyme filtrate into an aqueous solution of 0.1% glucose and 0.3% gluten in order to confirm the gluten degradation ability of the grain-fermented enzyme according to the present invention.
FIG. 6 shows results of confirming the gluten degradation ability of the grain-fermented enzyme using the Gliadin kit.

### [Detailed Description of Preferred Embodiments of Invention]

The first embodiment of the present invention relates to a grain-fermented enzyme fermented by *Bacillus amyloliquefaciens* strain, wherein the grains include one or more selected from the group consisting of defatted soybean, oat, brown rice, white bean, barley, sorghum, linseed, rice germ, pea, pea pod, wheat, rye and millet.

The strain may be *Bacillus amyloliquefaciens* NPKE6 deposited under accession number KCCM13257P, and the strain may have one or more selected from the group consisting of α-amylase activity, protease activity, gluten degradation activity and fibrinolytic enzyme activity, but it is not limited thereto.

The second embodiment of the present invention relates to a method for producing a grain-fermented enzyme, including the steps of: (a) soaking grains in water; (b) removing the soaking water; (c) steaming the grains, and then sterilizing them; (d) subdividing the sterilized grains, and then cooling them; (e) inoculating the subdivided grains with *Bacillus amyloliquefaciens* strain, and then mixing them; and (f) fermenting the grain inoculated with the strain.

The method for producing a grain-fermented enzyme according to the present invention may further include the step of hot air drying and powdering after step (f), but it is not limited thereto.

The hot air drying may be performed, for example, at 65 to 75 °C for 20 to 30 hours, preferably 22 to 26 hours, but it is not limited thereto. The powdering allows the enzyme to be formulated into different formulations as needed.

The grain may be one or more selected from the group consisting of defatted soybean, oat, brown rice, white bean, barley, sorghum, linseed, rice germ, pea, pea pod, wheat, rye and millet, but it is not limited thereto.

The strain may be *Bacillus amyloliquefaciens* NPKE6 deposited under accession number KCCM13257P, and the strain may have one or more selected from the group consisting of α-amylase activity, protease activity, gluten degradation activity and fibrinolytic enzyme activity, but it is not limited thereto.

The third embodiment of the present invention relates to a food composition including the grain-fermented enzyme. The food composition is preferably an enzyme food, but it is not limited thereto.

The food composition may include, in addition to the grain-fermented enzyme, two or more of fermented mixed grains selected from the group consisting of brown rice, white bean, sorghum and barley; a mixed powder of two or more grains selected from the group consisting of roasted brown rice, roasted soybean, and papaya extract; and potato extract powder, but it is not limited thereto.

The food composition may include 20 to 40% by weight ("wt%") of the grain-fermented enzyme, 50 to 70 wt% of the fermented mixed grain, 1 to 5 wt% of the grain mixed powder, and 1 to 5 wt% of the potato extract powder based on a total weight of the composition, but it is not limited thereto.

The food composition preferably includes 30 to 35 wt% of the grain-fermented enzyme, 55 to 65 wt% of the fermented mixed grain, 2 to 4 wt% of the grain mixed powder, and 2 to 4 wt% of the potato extract powder based on the total weight of the composition, but it is not limited thereto.

The food composition more preferably includes 33 to 34 wt% of the grain-fermented enzyme, 60 to 62 wt% of the fermented mixed grain, 2.5 to 3.5 wt% of the grain mixed powder, and 2.5 to 3.5 wt% of the potato extract powder based on the total weight of the composition, but it is not limited thereto.

Brown rice, which may be included in the fermented mixed grain, is rice harvested by drying and threshing the rice and removing the husk using a machine with rubber rollers, and is known to be effective in preventing adult diseases.

White bean, which may be included in the fermented mixed grain, is also called soybean, and in terms of nutrition, contains a lot of ingredients such as lecithin, saponin, isoflavone, and trypsin inhibitor. These ingredients have anti-cancer effects, decrease blood cholesterol, prevent obesity by inhibiting fat synthesis, activate intestinal motility through intestinal function, and prevent constipation by facilitating defecation.

Sorghum, which may be included in the fermented mixed grain, is low in fat and low in calories such that it is easily used as a diet food, and has effects of improving cholesterol levels and decomposing waste in the blood vessels. In addition, proanthocyanidin contained in the sorghum plays a role of strengthening immune function of the bladder and reducing oxidative stress in cells, thereby alleviating inflammation.

Barley, which may be included in the fermented mixed grains, is rich in dietary fiber to help with constipation, is rich in a polyphenol compound and helpful for improving antioxidant efficacy and immunity, and contains tocotrienols to decrease cholesterol levels, which in turn is possible to prevent vascular diseases.

Papaya extract, which may be included in the grain mixed powder, contains papain, a type of proteolytic enzyme extracted from the papaya fruit, wherein the papain is one of the powerful digestive enzymes found in nature, and is known to assist to activate digestive function, prevent stomach swelling, and have various effects such as promotion of diuresis, and anti-inflammatory activity.

In the potato extract powder included in the food composition, the potato is an alkaline food, protects the stomach by protecting and improving ulcers and mucous membranes due to excessive gastric acid, and contains a large amount of vitamin C (a similar amount to spinach or tangerines), wherein vitamin C in the potato is trapped in starch, making them relatively resistant to heat. Further, as compared to other vegetables or foods, the potato has a high content of potassium of 410 to 485 mg per 100 g, and the starch in the potato contains a large amount of pectin, a type of dietary fiber, thereby attaining effects of facilitating intestinal function and bowel movement.

The fourth embodiment of the present invention relates to the *Bacillus amyloliquefaciens* NPKE6 strain deposited under accession number KCCM13257P.

The strain may have α-amylase activity, protease activity, gluten degradation activity and fibrinolytic enzyme activity, but it is not limited thereto.

Hereinafter, in order to aid understanding of the present invention, it will be described in detail by way of examples, etc. However, the examples according to the present invention may be modified into various other forms, and the scope of the present invention should not be construed as being limited to the following examples. Examples of the present invention are provided to more completely describe the present invention to those skilled in the art.

### <Example 1> Identification of Bacillus amyloliquefaciens NPKE6 strain

### 1. Base sequence analysis of Bacillus amyloliquefaciens NPKE6 strain

DNA extraction and 16S rRNA sequence analysis for identification were performed by asking to Macrogen. Identification of bacteria was performed using the 16S rRNA sequence through the Basic Local Alignment Search Tool (BLAST) search engine of the National Center for Biotechnology Information (NCBI, www.ncbi.nlm.nih.gov).

As a result of analyzing the 16S rRNA base sequence for identification and classification of the isolated strain, the base sequence of SEQ ID NO. 1 was obtained.

### 2. Confirmation of biochemical characteristics of Bacillus amyloliquefaciens NPKE6 strain

The biochemical characteristics of *Bacillus amyloliquefaciens* NPKE6 strain were investigated using API 50CH (bioMerieux Co., France), which is used for Bacillus identification. The API kit was operated as follows according to the manufacturer's instructions for use, and results thereof are shown in Table 1 below.

*Bacillus amyloliquefaciens* NPKE6 strain was inoculated into TSB (17.0 g casein, 3.0 g soybean wheat, 5.0 g NaCl, 2.5 g dipotassium phosphate, 2.5 g dextrose, final pH 7.3 ± 0.2 at 25 °C) liquid medium. After culturing at 37 °C, the cells were dispensed into Eppendorf tubes and centrifuged at 10,000 rpm for 5 minutes, and then the cells were recovered and washed once with sterile saline solution (0.85%). The cells were suspended in sterile saline solution, inoculated into an ampoule of aseptically broken API 50CH medium, mixed evenly by pipetting, followed by dispensing each 150 µl into a micro tube of each test strip.

After completion of dispensing, a change in color was observed while culturing in an incubator at 37 °C for 24 hours, and then results of the observation were demonstrated by API kit. In Table 1 below, if each enzyme is used, it is indicated as +, and if it is not used, it is indicated as -.

**[TABLE 1]**

| Characteristic | Result | Characteristic | Result |
|---|---|---|---|
| Glycerol | - | Salicin | - |
| Erythritol | - | Cellobiose | - |
| D-arabinose | - | Maltose | - |
| L-arabinose | - | Lactose | - |
| Ribose | - | Melibiose | - |
| D-xylose | - | Sucrose | + |
| L-xylose | - | Trehalose | - |
| Adonitol | - | Inulin | - |
| Methyl-β-D-xylopyranoside | - | Melezitose | - |
| Galactose | - | Raffinose | - |
| Glucose | - | Starch | - |
| Fructose | - | Glycogen | - |
| Mannose | - | Xylitol | - |
| Sorbose | - | Gentibiose | - |
| Rhamnose | - | D-turanose | - |
| Dulcitol | - | D-lyxose | - |
| Inositol | - | D-tagatose | - |
| Mannitol | - | D-fucose | - |
| Sorbitol | - | L-fucose | - |
| Methyl-α-D-mannopyranoside | - | D-arabitol | - |
| Methyl-α-D-glucoside | - | L-arabitol | - |
| N-acetyl-glucosamine | - | Gluconate | - |
| Amygdalin | - | 2-keto-gluconate | - |
| Arbutin | - | 5-keto-gluconate | - |
| Esculin | + | | |

According to a result of the identification, as a result of combining the biochemical characteristics, the selected NPKE6 strain was identified as *Bacillus amyloliquefaciens* and named as *Bacillus amyloliquefaciens* NPKE6, which was deposited on October 31, 2022 at the Korean Culture Center of Microorganisms (KCCM) and given with accession number KCCM13257P.

### <Example 2> Production of grain-fermented enzyme using Bacillus amyloliquefaciens NPKE6 strain

### 1. Cultivation of strain

*Bacillus amyloliquefaciens* NPKE6 strain was inoculated into BM (D-Glucose monohydrate (13.5 g/L), Hydrolysed soy protein (3 g/L), Yeast extract (3 g/L), Sodium carbonate (0.3 g/L), Magnesium sulfate heptahydrate (0.2 g/L)) medium (1.0% v/v), followed by culturing at 30 °C for 24 hours.

### 2. Production of grain-fermented enzyme

After soaking oats in 1 volume of water for 16 hours, the water was removed and defatted soybean meal was mixed with water in 1 time volume of the defatted soybean meal. The mixture was steam sterilized at 100 °C for 3 hours, then subdivided into 2.5 kg portions on each tray and left to cool.

The *Bacillus amyloliquefaciens* NPKE6 seed cultured at 30 °C and 150 rpm was inoculated into BM medium at an inoculation rate of 2% (v/w), and then fermented for 24 hours under conditions of relative humidity of 80% or more.

After completion of fermentation, each tray was dried with hot air at 70 °C for 24 hours, and pulverized to a size of 60 mesh using a multi-purpose grinder thus to produce the grain-fermented enzyme.

### <Example 3> Enzyme activity analysis of grain-fermented enzyme

### 1. Confirmation of gluten degradation ability of NPKE6 strain

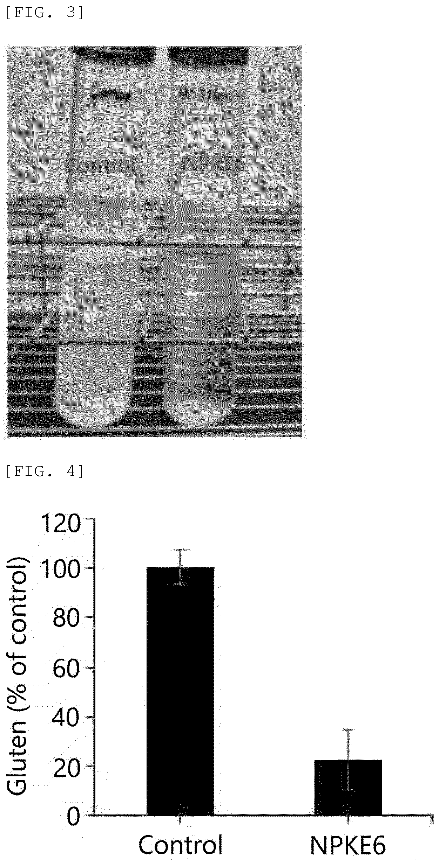

*Bacillus amyloliquefaciens* NPKE6 strain was inoculated into BM medium (1.0 % v/v) and cultured at 30 °C for 24 hours. As shown in FIG. 3, the culture medium was inoculated at 5% in an aqueous solution containing 0.1% glucose and 0.5% gluten, and the extent to which the gluten medium became transparent was visually confirmed after 24 hours. As shown in FIG. 4, using the RIDASCREEN Gliadin kit (R-Biopharm AG, Germany), gluten degradation ability in a gluten medium was determined thus to confirm that the gluten content decreased by about 77% compared to the control when inoculated with the NPKE6 strain.

### 2. Confirmation of gluten degradation ability of NPKE6 culture filtrate and grain-fermented enzyme filtrate

*Bacillus amyloliquefaciens* NPKE6 strain was inoculated into BM medium (1.0 % v/v) and cultured at 30 °C for 24 hours. The culture solution and 1 g of grain-fermented enzyme diluted with 10 mL of sterile water were centrifuged at 4000 rpm for 20 minutes and filtered through a 0.2 µm filter to prepare a culture filtrate and a grain-fermented enzyme filtrate.

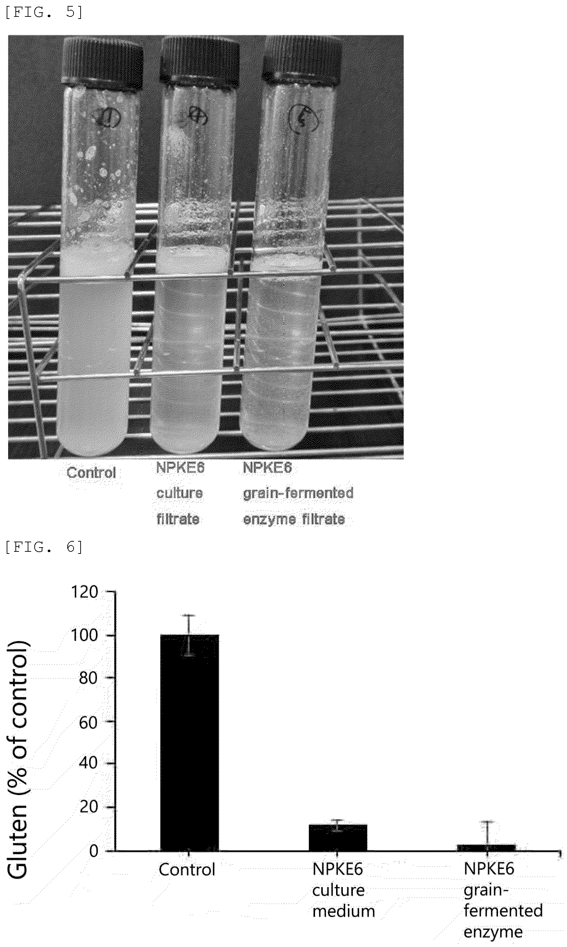

As shown in FIG. 5, the filtered culture filtrate and grain-fermented enzyme filtrate were inoculated at 5% in 0.1% glucose and 0.3% gluten aqueous solutions, respectively, and the extent to which the gluten medium became transparent was visually confirmed after 24 hours. As shown in FIG. 6, using the RIDASCREEN Gliadin kit (R-Biopharm AG, Germany), gluten degradation ability in a gluten medium was determined thus to confirm that the gluten content was decreased by about 89% compared to the control when inoculated with the strain filtrate, in addition, to confirm that the gluten content was decreased by about 98% compared to the control when inoculated with the grain-fermented enzyme filtrate, respectively.

As the specific parts of the present invention have been described in detail above, it will be obvious to those skilled in the art that these specific techniques are merely preferred embodiments and do not limit the scope of the present invention.

Accordingly, the substantial scope of the present invention will be defined by the appended claims and their equivalents. Simple modifications or changes of the present invention could be easily used by those skilled in the art, and all such modifications or changes could be considered to be included in the scope of the present invention.

### [Accession number]

Name of depository organization: Korean Culture Center of Microorganisms (KCCM)
Address of depository organization: Yurim Building, 45 Hongjenae 2 ga-gil, Seodaemun-gu, Seoul
Date of deposit: October 31, 2022
Deposit number: KCCM13257P

### Budapest Treaty on the International Recognition of the Deposit of Microorganisms for the Purposes of Patent Procedure

### International Form

Depositor: Naturepure Korea Co., Ltd. Receipt issued pursuant to Rule 7.1 by 61 Ecogil, Damyang-eup, Damyang-gun the International Depositary Authority Jeollanamdo, Korea

| I. Identification of the Microorganism | |
|---|---|
| Identification reference given by the Depositor: *Bacillus amyloliquefaciens* NPKE6 | Accession number given by the International Depositary Authority: KCCM13257P |

| II. Scientific description and/or Proposed Taxonomic Designation | |
|---|---|
| The microorganism identified under I above was accompanied by: | |
| [ ] a scientific description | |
| [ ] a proposed taxonomic designation | |
| (Mark with a cross (x) where applicable) | |

| III. Receipt and Acceptance | |
|---|---|
| This International Depositary Authority accepts the microorganism identified under I above, which was received on October 31, 2022. | |

| IV. Receipt or Request for Conversion | |
|---|---|
| The microorganism identified under I above was received by this International Depositary Authority, and a request to convert the original deposit to a deposit under the Budapest Treaty was received by it. | |

| V. International Depositary Authority | |
|---|---|
| Name: Korean Culture Center of Microorganisms | Signature(s) of person(s) having the power to represent the International Depositary Authority or of authorized official(s): |
| Address: Yurim Building, 45 Hongjenae 2 ga-gil, Seodaemun-gu, Seoul | |
| | Date: October 31, 2022 |

| | |
|---|---|
| * We sincerely declare that the above is a true and correct translation of the original text. | |

## Claims

1. A grain-fermented enzyme fermented by *Bacillus amyloliquefaciens* strain,
wherein the grain is one or more selected from the group consisting of defatted soybean, oat, brown rice, white bean, barley, sorghum, linseed, rice germ, pea, pea pod, wheat, rye and millet.

2. The grain-fermented enzyme according to claim 1, wherein the strain is *Bacillus amyloliquefaciens* NPKE6 deposited under accession number KCCM13257P.

3. The grain-fermented enzyme according to claim **1,** wherein the grain-fermented enzyme has α-amylase activity, protease activity, gluten degradation activity and fibrinolytic enzyme activity.

4. A method for producing a grain-fermented enzyme, comprising:
(a) soaking grains in water;
(b) removing the soaking water;
(c) steaming the grains, and then sterilizing them;
(d) subdividing the sterilized grains, and then cooling them;
(e) inoculating the subdivided grains with *Bacillus amyloliquefaciens* strain, and then mixing them; and
(f) fermenting the grain inoculated with the strain.

5. The method for producing a grain-fermented enzyme according to claim 4, further comprising the step of hot air drying and powdering after step (f).

6. The method for producing a grain-fermented enzyme according to claim 4, wherein the grain is one or more selected from the group consisting of defatted soybean, oat, brown rice, white bean, barley, sorghum, linseed, rice germ, pea, pea pod, wheat, rye and millet.

7. The method for producing a grain-fermented enzyme according to claim 4, wherein the strain is *Bacillus amyloliquefaciens* NPKE6 deposited under accession number KCCM13257P.

8. A food composition comprising the grain-fermented enzyme according to any one of claims 1 to 3.

9. The food composition according to claim 8, wherein the food composition is an enzyme food.

10. *Bacillus amyloliquefaciens* NPKE6 strain deposited under the accession number KCCM13257P.

11. The *Bacillus amyloliquefaciens* NPKE6 strain according to claim 10, wherein the strain has α-amylase activity, protease activity, gluten degradation activity and fibrinolytic enzyme activity.
